(19) Europäisches Patentamt
European Patent Office
Office européen des brevets

(11) **EP 4 011 284 A1**

(12) **EUROPEAN PATENT APPLICATION**

(43) Date of publication:
**15.06.2022 Bulletin 2022/24**

(21) Application number: **20213169.4**

(22) Date of filing: **10.12.2020**

(51) International Patent Classification (IPC):
**A61B 5/02** *(2006.01)*    **A61B 5/022** *(2006.01)*
**A61B 5/021** *(2006.01)*    **A61B 5/00** *(2006.01)*

(52) Cooperative Patent Classification (CPC):
**A61B 5/02225; A61B 5/02007;** A61B 5/02141;
A61B 5/7235

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **Koninklijke Philips N.V.
5656 AG Eindhoven (NL)**

(72) Inventors:
• **BOGATU, Laura Ioana**
  **5656 AE Eindhoven (NL)**
• **MUEHLSTEFF, Jens**
  **5656 AE Eindhoven (NL)**
• **WOERLEE, Pierre**
  **5656 AE Eindhoven (NL)**

(74) Representative: **Philips Intellectual Property &
Standards
High Tech Campus 52
5656 AG Eindhoven (NL)**

(54) **METHOD AND APPARATUS FOR DETERMINING INFORMATION ABOUT AN ARTERIAL PROPERTY OF A SUBJECT**

(57)    According to an aspect, there is provided a method for determining information about an arterial property of an artery of a subject. The method comprises generating (103) volume oscillations at a first oscillation frequency in a first cuff that is at a first location on a limb of the subject, wherein the volume oscillations are generated at a plurality of cuff pressures in the first cuff; measuring (105) the pressure in the first cuff during the generation of the volume oscillations to produce a first cuff pressure signal; determining (107) first arterial volume responses representing a volume response of the artery to the volume oscillations at respective cuff pressures, wherein the first arterial volume responses are determined based on the first cuff pressure signal, a cuff compliance of the first cuff, and a volume oscillation signal related to the first oscillation frequency of the volume oscillations generated in the first cuff; and determining (109) the information about the arterial property of the subject from the first arterial volume responses.

Fig. 5

**Description**

FIELD OF THE INVENTION

This disclosure relates to a method and apparatus for determining information about an arterial property of a subject, and in particular to determining this information non-invasively.

BACKGROUND OF THE INVENTION

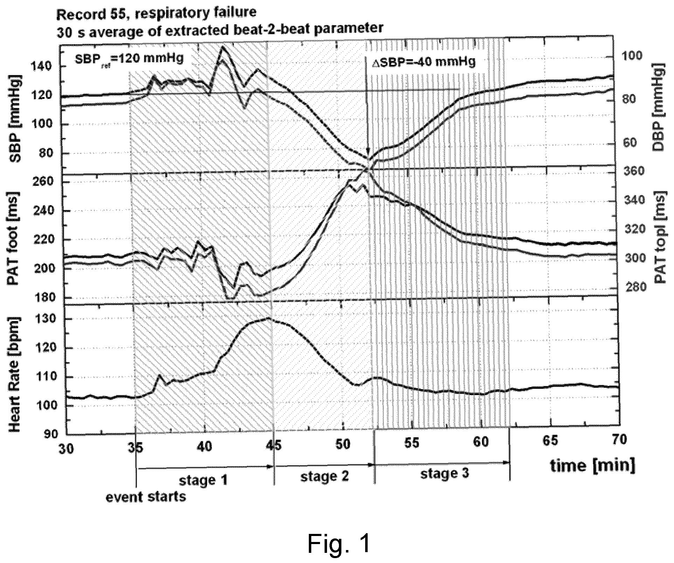

Early detection of critical states of a patient's condition is a key need in a clinical setting, such as a hospital. Typically used signals such as heart rate can be very unspecific or late in indicating changes in a patient's condition (e.g. blood pressure (BP)).For example, a hypotension (low blood pressure) phase can be detected late when looking at blood pressure only. This is illustrated with reference to Fig. 1. The plots in Fig. 1 relate to a hypotensive phase of a patient in an intensive care unit (ICU) reaching a critical low systolic BP (SBP) of 80 mmHg. The systolic BP over time is shown in the top part of Fig. 1.However, the heart rate (shown in the bottom part of Fig. 1) responds early but non-specifically by increasing in order compensate for decrease in BP. The pulse arrival time (PAT) (plotted in the middle part of Fig. 1) in this case changes due to changes in BP, but its change does not predict BP.

Therefore there is a need for new or better measures that respond quickly, sensitively and specifically to patient conditions that can be monitored or derived easily and unobtrusively for the patient.

An underestimated and underutilized source of information in current clinical practice is the vascular (arterial) compliance, which plays a fundamental role in the integrity of the circulatory system. Arterial compliance adjusts with respect to changes in blood pressure, circadian rhythms, physical activity, stress, as well as with respect to longer term changes caused by age, lifestyle, etc. Compliance, and in particular arterial compliance, can reveal a considerable amount of information regarding the health status of a patient or subject. Conventionally arterial compliance is measured using imaging solutions such as vascular ultrasound or MRI (magnetic resonance imaging), but this makes it unfeasible to continuously measure arterial compliance for most patients (e.g. those in an ICU).

Besides the arterial compliance, or the elasticity properties of the arterial wall (which enable constriction/distension), new regulation mechanisms of the circulatory system have been discovered, e.g. non-linearity properties of the arterial wall, viscosity, damping properties, existence of a cut-off frequency, and strain-dependent activation of the smooth muscle tone. Because of this, methods for observing and measuring these compensatory mechanisms are being developed. However, until now, proposed methods for obtaining such complex regulation measurements involve invasive measurements, ex-vivo studies of arteries, or inference via complex physiological models involving uncertainties and assumptions. Some of those methods are described in "Arterial wall mechanics as a function of heart rate: role of vascular smooth muscle" by Fernando Pablo Salvucci et al., 2007 J. Phys.: Conf. Ser. 90 012010, and "Beat to Beat Modulation of Arterial Pulse Wave Velocity Induced by Vascular Smooth Muscle Tone" by L. J. Cymberknop et al., 2019 41st Annual International Conference of the IEEE Engineering in Medicine and Biology Society (EMBC), Berlin, Germany, 2019, pp. 5030-5033.

The arterial compliance represents the ability of arteries to distend and contract in response to changes in pressure. Consequently, arterial compliance (denoted $C_a$) can be expressed as shown in Eq. 1 below.

$$C_a(P_{tm}) = \frac{dV_{art}}{dP_{tm}} \qquad (1)$$

In Eq. 1 $V_{art}$ is the arterial volume and $P_{tm}$ is the transmural pressure across the arterial wall. The transmural pressure $P_{tm}$ is given by Eq. 2:

$$P_{tm} = P_{art} - P_{ext} \qquad (2)$$

where $P_{art}$ is the arterial blood pressure and $P_{ext}$ is pressure outside the artery (e.g. atmospheric pressure, or the pressure inside a cuff (referred to as 'cuff pressure'), if a cuff is placed on the arm or limb. The maximum of $P_{art}$ is the systolic blood pressure and the minimum of $P_{art}$ is the diastolic blood pressure (DBP). $C_a$, the arterial compliance, is a highly nonlinear and subject-specific function of the transmural pressure $P_{tm}$ (i.e. the pressure across the arterial wall).

In principle, arterial volume can be measured by means of high-resolution imaging (e.g. ultrasound), which can reveal changes in arterial diameter as arterial pressure varies between systolic and diastolic values. In practice however, difficulties are caused by the fact that the procedure is not automatic - it requires highly skilled operators and is prone to errors (as noted in "Venous occlusion plethysmography in cardiovascular research: methodology and clinical applications" by Wilkinson IB and Webb DJ, Br. J. Clin. Pharmacol. 2001;52:631-646).Besides this, if information about arterial properties is required over a wider range of transmural pressures, then imaging technologies would have to be incorporated into cuff setups, further complicating the measurement.

A different approach to arterial volume measurements involves the use of the already existing BP cuff (i.e. a cuff used when measuring blood pressure).The pressure oscillations inside the cuff are representative of arterial volume changes due to the pulsating nature of systemic blood pressure. However, in order to extract arterial properties from this signal, accurate knowledge of arterial pressure is necessary. Such accuracy can be obtained invasively via an arterial line. For a non-invasive measurement, the blood pressure value can be obtained via typical cuff-based oscillometric measurements. However, the oscillometric measurement has been found to be inaccurate in certain patient groups (such as hypo- and hypertensive patients). In addition to this, especially in hemodynamically unstable patients, BP can change within a very short time span, and there are beat-to-beat variations caused by breathing. Therefore, measuring blood pressure at one time and arterial volume change at a different time might not give a correct arterial property value.

There are several ways in which estimated arterial compliance or viscosity properties can be used for the purpose of better detection of patient deterioration. The most straightforward approach is the quantification of changes in arterial compliance, arterial collapse or viscosity values. These changes can be made available to clinicians via an additional score displayed on an operating room (OR)/ICU monitor in a similar way to heart and blood pressure values. Another method is based on the incorporation of arterial compliance and viscosity information into physiological models for the purpose of approximating system wide parameters such as cardiac output or peripheral resistance.

Knowledge of arterial compliance can provide a more continuous non-invasive BP estimation by enabling accurate calibration of BP surrogates. For example, PAT can be a good indicator of changes in systolic blood pressure, as shown in Fig. 1.To make use of this, a calibration step is required to link PAT changes to BP changes. A good approximation of arterial compliance can bring multiple benefits to this calibration step in terms of accuracy and range of transmural pressures considered in the BP-PAT function. In addition, information about arterial compliance reduces the need for empirical assumptions in the calibration of BP surrogates and supports more physiology-based interpretations.

A related application is the assessment of endothelial function - the ability of the arteries to adjust lumen size in order to maintain homeostasis. Usually this assessment is performed by means of measuring artery dimensions following vascular occlusion of several minutes (this is known as flow-mediated dilation).The short-term changes in flow produce changes in arterial lumen size, revealing information about the stability of a patient (such as the presence of sepsis), or progression of cardiovascular diseases. Ultrasound, photoplethysmogram (PPG), or PAT-based techniques are currently employed in the assessment of artery dimension adjustments, such as in "Flow-mediated dilation and cardiovascular risk prediction: a systematic review with meta-analysis" by Ras, R. T. et al., Int J Cardiol 168, 344-351, "Influence of respiratory rate on the variability of blood volume pulse characteristics" by Selvaraj N, et al., J Med Eng Technol. 2009;33(5):370-375, "Assessment of Vascular Health With Photoplethysmographic Waveforms From the Fingertip" by Wu HT et al., IEEE J Biomed Health Inform. 2017 Mar; 21(2):382-386, and "Finger photoplethysmogram pulse amplitude changes induced by flow-mediated dilation" by Zahedi E, Physiol Meas. 2008;29:625-37.These current techniques face limitations, for example the obvious challenges relating to the imaging of arteries, while other PPG and PAT based techniques cannot overcome the fact that occlusion needs to take place for several minutes to produce a detectable change in artery size (which is traumatic for the patient).Information about compliance or viscosity could bring benefits to endothelial response evaluation by potentially enabling more accurate and comfortable (faster) measurements of changes in artery size compared to current practices.

Therefore, arterial properties such as arterial compliance and/or arterial viscosity are useful in monitoring a health status of a subject. It is therefore desirable to provide improved ways to non-invasively estimate information about these and other arterial properties of a subject.

SUMMARY OF THE INVENTION

A solution to non-invasively measure arterial properties can be developed from techniques for measuring blood pressure using external perturbations (known as external pressure non-invasive BP (NIBP) measurements).With an external oscillation configuration, the arterial volume can be measured as a response to external, well-defined pressure changes that result in changes in transmural pressure. In this case, knowledge of the internal arterial pressure is not necessary. Information about the arterial compliance can be derived from this arterial volume response. In addition, the arterial response to different frequencies (beyond heart rate) can be measured via external perturbations. This can provide information regarding viscosity properties of the artery.

According to a first specific aspect, there is provided a method for determining information about an arterial property of an artery of a subject. The method comprises (a) generating volume oscillations at a first oscillation frequency in a first cuff that is at a first location on a limb of the subject, wherein the volume oscillations are generated at a plurality of cuff pressures in the first cuff; (b) measuring the pressure in the first cuff during the generation of the volume oscillations to produce a first cuff pressure signal; (c) determining first arterial volume responses representing a volume response of the artery to the volume oscillations at respective cuff pressures, wherein the first arterial volume responses are determined based on the first cuff pressure signal, a cuff compliance of the first cuff, and a volume oscillation signal related to the first oscillation frequency of the volume oscillations generated in the first cuff; and (d) determining the information about the arterial property of the subject from the first arterial volume responses. This aspect has the advantage that an arterial property can be measured non-invasively without requiring knowledge of the internal arterial pressure.

In some embodiments, step (c) comprises determining a first arterial volume response using $dV_{art} = (P_{cuff} - P_{abs}) * C_{cuff} - dV_{osc}$, where $dV_{art}$ is the first arterial volume response, $P_{cuff}$ is the first cuff pressure signal, $P_{abs}$ is respective cuff pressure in the first cuff, $C_{cuff}$ is the cuff compliance of the first cuff and $dV_{osc}$ is the signal representing the change in volume in the cuff due to the generated volume oscillations.

In some embodiments, step (c) further comprises band-pass filtering the first cuff pressure signal to obtain signal components corresponding to the first oscillation frequency of the volume oscillations; and determining the first arterial volume response based on the band-pass filtered first cuff pressure signal. These embodiments provide the advantage that signal components due to heart beats are not taken into account when determining the first arterial volume response.

In some embodiments, the first oscillation frequency of the volume oscillations is set to a frequency different to the heart rate of the subject. In these embodiments, the first oscillation frequency of the volume oscillations can be in the range of 1 Hz to 3Hz. These embodiments enable the volume oscillations due to heart beats to be filtered out.

In alternative embodiments, the method can further comprise, prior to step (a), inflating a second cuff that is at a second location on the subject to a blocking cuff pressure that is a cuff pressure above the systolic blood pressure of the subject, wherein the second location is on the same limb as, and upstream of, the first location; and steps (a) and (b) are performed while the second cuff is inflated to the blocking cuff pressure. These embodiments have the advantage that volume oscillations due to blood flow are stopped, which makes the subsequent processing of the first cuff pressure signal more straightforward. In these embodiments, step (d) can comprise determining a function of arterial volume response against transmural pressure from the first arterial volume response; and determining the information about the arterial property from the determined function.

In some embodiments, the arterial property comprises arterial compliance. In these embodiments, the method can further comprise: determining the cuff pressure in the first cuff at which a maximum arterial compliance occurs from the function of arterial volume response against transmural pressure; and determining a mean systemic filling pressure, MSFP, for the subject as the cuff pressure in the first cuff at which the maximum arterial compliance occurs. These embodiments provide a simple approach to non-invasively determining MSFP without having to measure the arterial blood pressure.

In some embodiments, the method further comprises: (e) repeating steps (a) and (b) for one or more further oscillation frequencies for the volume oscillations to produce a respective one or more further cuff pressure signals; (f) determining further arterial volume responses for each of the one or more further oscillation frequencies, the further arterial volume responses representing a volume response of the artery to the volume oscillations at respective cuff pressures, wherein an arterial volume response is determined based on the respective further cuff pressure signal, the cuff compliance of the first cuff, and a respective volume oscillation signal related to the respective oscillation frequency of the volume oscillations generated in the first cuff; and step (d) comprises determining the information about the arterial property of the subject from the first arterial volume responses and the further arterial volume responses. These embodiments have the advantage that they provide information on the arterial volume response at different frequencies.

In these embodiments, steps (a), (b) and (e) can comprise (g) inflating the first cuff to a first cuff pressure; (h) generating volume oscillations at the first oscillation frequency in the first cuff and measuring the pressure in the first cuff during the generation of the volume oscillations; (i) repeating step (h) for one or more further oscillation frequencies in the first cuff; (j) inflating the first cuff to a second pressure; and (k) repeating steps (h) and (i) while the first cuff is inflated to the second pressure. Thus, these embodiments provide that information on the arterial volume response at different frequencies can be obtained from a single cuff inflation procedure.

In the above embodiments, the arterial property can be viscosity of the artery. In these embodiments, step (d) can comprise determining the information about the viscosity of the artery from a comparison of the first arterial volume responses and the further arterial volume responses.

In the above embodiments, the first oscillation frequency and the one or more further oscillation frequencies can be frequencies in the range of 1 Hz to 25 Hz.

In some embodiments, the volume oscillations at the first oscillation frequency have an amplitude in the range of 5 milliliters, ml, to 20 ml.

In some embodiments, the method further comprises determining the cuff compliance of the first cuff. In some embodiments, the cuff compliance can be determined by, prior to step (c): generating volume oscillations at a second oscillation frequency in the first cuff when the first cuff is inflated to a third cuff pressure; measuring the pressure in the first cuff during the generation of the volume oscillations at the second oscillation frequency to produce a third cuff pressure signal; band-pass filtering the third cuff pressure signal to obtain signal components corresponding to an oscillation frequency of the second volume oscillations; and determining the cuff compliance of the first cuff from the filtered third cuff pressure signal. These embodiments have the advantage that cuff compliance does not need to be known prior to the above method being performed, and also have the advantage that the method can be performed with any particular cuff (for example with the size of the cuff being determined according to the age/size of the subject).

In these embodiments, the step of determining the cuff compliance can comprise dividing the amplitude of the volume oscillations at the second oscillation frequency by the amplitude of the filtered third cuff pressure signal. In these embodiments, the amplitude of the volume oscillations at the second oscillation frequency can be less than the amplitude of the volume oscillations at the first oscillation frequency. In these embodiments, the amplitude of the volume oscillations at the second oscillation frequency can be in the range of 0.1 milliliters, ml, to 5 ml.

According to a second aspect, there is provided a computer program product comprising a computer readable medium having computer readable code embodied therein, the computer readable code being configured such that, on execution by a suitable computer or processing unit, the computer or processing unit is caused to perform the method according to the first aspect or any embodiment thereof. In some embodiments, the computer or processing unit is configured to connect to or interface with a pump or other oscillator component in order to perform step (a) and/or to connect to or interface with a cuff pressure sensor in order to perform step (b).

According to a third specific aspect, there is provided an apparatus for determining information about an arterial property of an artery of a subject. The apparatus is configured to (a) initiate the generation of volume oscillations at a first oscillation frequency in a first cuff that is at a first location on a limb of the subject, wherein the volume oscillations are to be generated at a plurality of cuff pressures in the first cuff; (b) obtain a first cuff pressure signal that comprises measurements of the pressure in the first cuff during the generation of the volume oscillations; (c) determine first arterial volume responses representing a volume response of the artery to the volume oscillations at respective cuff pressures, wherein the first arterial volume responses are determined based on the first cuff pressure signal, a cuff compliance of the first cuff, and a volume oscillation signal related to the first oscillation frequency of the volume oscillations generated in the first cuff; and (d) determine the information about the arterial property of the subject from the first arterial volume responses. This aspect has the advantage that an arterial property can be measured non-invasively without requiring knowledge of the internal arterial pressure.

In some embodiments, operation (c) comprises determining a first arterial volume response using $dV_{art} = (P_{cuff} - P_{abs}) * C_{cuff} - dV_{osc}$, where $dV_{art}$ is the first arterial volume response, $P_{cuff}$ is the first cuff pressure signal, $P_{abs}$ is respective cuff pressure in the first cuff, $C_{cuff}$ is the cuff compliance of the first cuff and $dV_{osc}$ is the signal representing the change in volume in the cuff due to the generated volume oscillations.

In some embodiments, operation (c) further comprises band-pass filtering the first cuff pressure signal to obtain signal components corresponding to the first oscillation frequency of the volume oscillations; and determining the first arterial volume response based on the band-pass filtered first cuff pressure signal. These embodiments provide the advantage that signal components due to heart beats are not taken into account when determining the first arterial volume response.

In some embodiments, the first oscillation frequency of the volume oscillations is set to a frequency different to the heart rate of the subject. In these embodiments, the first oscillation frequency of the volume oscillations can be in the range of 1 Hz to 3Hz. These embodiments enable the volume oscillations due to heart beats to be filtered out.

In alternative embodiments, the apparatus can be further configured to, prior to operation (a), initiate the inflation of a second cuff that is at a second location on the subject to a blocking cuff pressure that is a cuff pressure above the systolic blood pressure of the subject, wherein the second location is on the same limb as, and upstream of, the first location; and operations (a) and (b) are performed while the second cuff is inflated to the blocking cuff pressure. These embodiments have the advantage that volume oscillations due to blood flow are stopped, which makes the subsequent processing of the first cuff pressure signal more straightforward. In these embodiments, operation (d) can comprise determining a function of arterial volume response against transmural pressure from the first arterial volume response; and determining the information about the arterial property from the determined function.

In some embodiments, the arterial property comprises arterial compliance. In these embodiments, the apparatus can be further configured to: determine the cuff pressure in the first cuff at which a maximum arterial compliance occurs from the function of arterial volume response against transmural pressure; and determine a mean systemic filling pressure, MSFP, for the subject as the cuff pressure in the first cuff at which the maximum arterial compliance occurs. These embodiments provide a simple approach to non-invasively determining MSFP without having to measure the arterial blood pressure.

In some embodiments, the apparatus is further configured to: (e) repeat operations (a) and (b) for one or more further oscillation frequencies for the volume oscillations to produce a respective one or more further cuff pressure signals; (f) determine further arterial volume responses for each of the one or more further oscillation frequencies, the further arterial volume responses representing a volume response of the artery to the volume oscillations at respective cuff pressures, wherein an arterial volume response is determined based on the respective further cuff pressure signal, the cuff compliance of the first cuff, and a respective volume oscillation signal related to the respective oscillation frequency of the volume oscillations generated in the first cuff; and operation (d) comprises determining the information about the arterial property of the subject from the first arterial volume responses and the further arterial volume responses. These embodiments have the advantage that they provide information on the arterial volume response at different frequencies.

In these embodiments, operations (a), (b) and (e) can comprise (g) initiating the inflation of the first cuff to a first cuff pressure; (h) initiating the generation volume oscillations at the first oscillation frequency in the first cuff and measuring the pressure in the first cuff during the generation of the volume oscillations; (i) repeating operation (h) for one or more further oscillation frequencies in the first cuff; (j) initiating the inflation of the first cuff to a second pressure; and (k) repeating operations (h) and (i) while the first cuff is inflated to the second pressure. Thus, these embodiments provide that information on the arterial volume response at different frequencies can be obtained from a single cuff inflation procedure.

In the above embodiments, the arterial property can be viscosity of the artery. In these embodiments, operation (d) can comprise determining the information about the viscosity of the artery from a comparison of the first arterial volume responses and the further arterial volume responses.

In the above embodiments, the first oscillation frequency and the one or more further oscillation frequencies can be frequencies in the range of 1 Hz to 25 Hz.

In some embodiments, the volume oscillations at the first oscillation frequency have an amplitude in the range of 5 milliliters, ml, to 20 ml.

In some embodiments, the apparatus is further configured to determine the cuff compliance of the first cuff. In some embodiments, the cuff compliance can be determined by, prior to operation (c): initiating the generation of volume oscillations at a second oscillation frequency in the first cuff when the first cuff is inflated to a third cuff pressure; obtain a third cuff pressure signal that comprises measurements of the pressure in the first cuff during the generation of the volume oscillations at the second oscillation frequency; band-pass filtering the third cuff pressure signal to obtain signal components corresponding to an oscillation frequency of the second volume oscillations; and determining the cuff compliance of the first cuff from the filtered third cuff pressure signal. These embodiments have the advantage that cuff compliance does not need to be known prior to the above process being performed, and also have the advantage that the process can be performed with any particular cuff (for example with the size of the cuff being determined according to the age/size of the subject).

In these embodiments, the apparatus can be configured to determine the cuff compliance by dividing the amplitude of the volume oscillations at the second oscillation frequency by the amplitude of the filtered third cuff pressure signal. In these embodiments, the amplitude of the volume oscillations at the second oscillation frequency can be less than the amplitude of the volume oscillations at the first oscillation frequency. In these embodiments, the amplitude of the volume oscillations at the second oscillation frequency can be in the range of 0.1 milliliters, ml, to 5 ml.

These and other aspects will be apparent from and elucidated with reference to the embodiment(s) described hereinafter.

The above embodiments thus provide for improved ways to determine information about arterial properties (such as arterial compliance and viscosity of the artery) non-invasively, that can be performed using existing equipment or apparatus that is used for patient monitoring. Detection of changes in actively regulated arterial properties can reveal activation of compensatory regulation mechanisms in the body, for example preceding a hypotensive event. Further, as the above embodiments use external pressure oscillations, the improved information about arterial properties is obtained without requiring a measurement of arterial blood pressure (whether invasively or non-invasively).

Information about an arterial property can be used for early detection of patient deterioration, since changes in arterial viscoelastic properties could precede large changes in blood pressure and hemodynamic instability. Information about an arterial property can also or alternatively be used for diagnosis and treatment of cardiovascular diseases, as measurement of arterial viscoelastic properties could play a role in the assessment of cardiovascular disease progression and in the evaluation of responsiveness to treatment. Information about an arterial property can also or alternatively be used for achieving improved accuracy in the calibration of blood pressure surrogate measures such as pulse arrival time and pulse morphology features. Furthermore, an improved and optionally continuous measurement of information about arterial properties can enable the development of personalized medical procedures and interventions, since arterial viscoelasticity is an important factor in better identifying different patient groups, e.g. related to medication responses.

BRIEF DESCRIPTION OF THE DRAWINGS

Exemplary embodiments will now be described, by way of example only, with reference to the following drawings, in which:

Fig. 1 is a graph illustrating measurements of heart rate, pulse arrival time and systolic blood pressure for a subject experiencing a hypotensive phase;

Fig. 2 is a block diagram of an apparatus that can be used to implement the techniques described herein;

Fig. 3 is an illustration of an exemplary measurement setup on a subject according to an embodiment;

Fig. 4 is a graph illustrating arterial volume oscillations under the cuff as the cuff inflates;

Fig. 5 is a flow chart illustrating a method of determining information about one or more arterial properties according to various embodiments;

Fig. 6 shows a graph illustrating changes in arterial pressure over time and a graph illustrating artificial volume oscillations generated inside the cuff over time;

Fig. 7(a) is a graph illustrating a cuff pressure signal obtained from a cuff that is subject to the volume oscillations shown in Fig. 6(b) when the arterial flow under the cuff is not blocked and Fig. 7(b) is a graph illustrating a cuff pressure signal obtained from a cuff that is subject to the volume oscillations shown in Fig. 6(b) when the blood flow is blocked using another cuff;

Fig. 8 illustrates an arterial volume response obtained from the cuff pressure signal in Fig. 7(a);

Fig. 9 shows a part of the graph in Fig. 8 in more detail;

Fig. 10 shows a part of the graph in Fig. 9 in more detail; and

Fig. 11 is a graph illustrating arterial volume as a function of transmural pressure.

## DETAILED DESCRIPTION OF EMBODIMENTS

As noted above, arterial properties of a subject, such as arterial compliance and/or arterial viscosity, are useful for monitoring the health status of a subject. These properties can be useful for the early detection of deterioration of the health of the subject, for example where changes in these properties precede changes in more regularly monitored properties such as heart rate and blood pressure. These properties can also be useful in the diagnosis and treatment of cardiovascular diseases, and/or they can be used in calibrating blood pressure surrogate measures.

Embodiments of the techniques described herein provide for a more accurate non-invasive measurement of arterial compliance. The techniques do not require knowledge of blood pressure, which is typically the most significant source of error in the measurement of arterial properties. Embodiments of the techniques described herein provide for a non-invasive measurement of arterial viscosity. Conventionally, measurement of arterial viscosity is only performed invasively, via ex-vivo measurements, or through inference via physiological models. Further embodiments provide for a non-invasive measurement of mean systemic filling pressure (MSFP) using the measurements of arterial compliance.

The techniques are based on a new understanding of how a cuff typically used in blood pressure measurements can be used to both apply a well-defined external pressure to an artery, and to simultaneously measure arterial volume response to the external pressure change without the need for an additional volume sensor, such as an ultrasound sensor. In the following, various parameters are used to describe and illustrate the invention. These parameters are defined in Table 1 below for convenience.

Table 1

| $C_a$ | Arterial compliance |
|---|---|
| $V_{art}$ | Arterial volume |
| $Ptm$ | Transmural pressure across the arterial wall |
| $dV_{art}$ | Change in arterial volume |
| $dPtm$ | Change in transmural pressure across the arterial wall |
| $P_{art}$ | Arterial blood pressure |
| $Pext$ | Pressure outside the artery |
| $P_{abs}$ | Absolute pressure |
| $dV_{osc}$ | Change in cuff oscillator volume |
| $C_{cuff}$ | Cuff compliance |
| $dP_{osc}$ | Pressure response of the cuff to volume oscillations |
| $d_{Part}$ | Pressure response of the artery to volume oscillations |
| $P_{cuff}$ | Pressure measured in the cuff |

(continued)

| $V_{osc}$ | Oscillator volume |
|---|---|

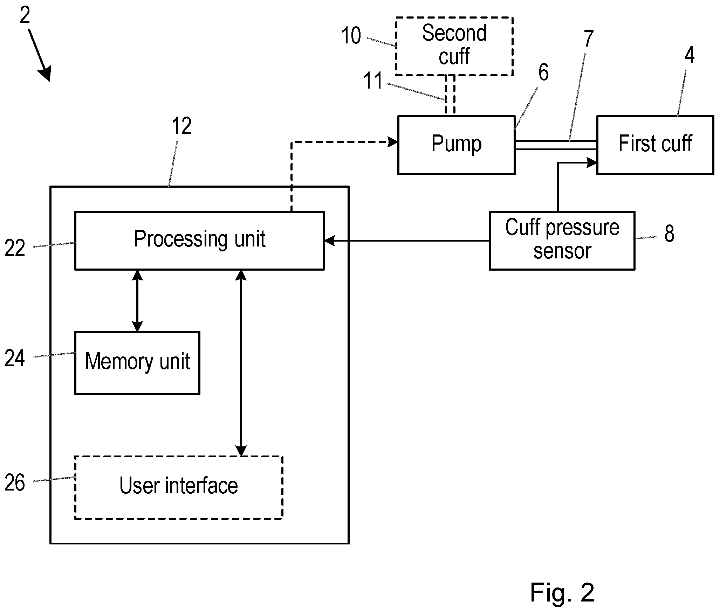

Fig. 2 shows a block diagram of a system 2 that can be used for estimating an arterial property according to various embodiments of the techniques described herein. The system 2 comprises a cuff 4 (which is also referred to as 'first cuff 4'), a pump 6 that is connected to the cuff 4 (e.g. via a connecting tube 7) and a cuff pressure sensor 8 for measuring the pressure in the cuff 4.The cuff 4 is to be placed around a body part of a subject of interest, e.g. around a limb such as an arm or leg, and the pump 6 is controllable to selectively inflate the cuff 4.The pump 6 may also be able to selectively deflate the cuff 4, and/or a valve (not shown) may be provided for enabling the cuff 4 to be selectively deflated.

While the cuff 4 is inflated to some pressure level(s), volume oscillations are generated inside the cuff 4.For example, volume oscillations can be generated by adding and then removing air from the cuff 4.In some embodiments, the pump 6 is able to generate the volume oscillations inside the cuff 4.In alternative embodiments, the system 2 can include a separate oscillator component (not shown in Fig. 2) that is connected to the pump 6, the cuff 4 and/or the connecting tube 7 and that is used to generate the volume oscillations inside the cuff 4.As described further below, the magnitude of the volume oscillations are small relative to the volume of the cuff 4 (e.g. the oscillations may have an amplitude of up to 25 milliliters (ml), with a typical inflated volume of a cuff 4 being in the range of 100 ml and 300 ml.

The cuff pressure sensor 8 measures the pressure in the cuff 4, at least when the cuff 4 is applying a stimulus on the artery located under the cuff 4, e.g. during inflation of the cuff 4, during deflation of the cuff 4, and/or while the pressure in the cuff 4 is held at a particular level, and while the pump 6 is generating volume oscillations in the cuff 4.The cuff pressure sensor 8 outputs a cuff pressure signal that represents, or relates to, measurements of the pressure in the cuff 4 over time. The cuff pressure signal is denoted $P_{cuff}$, and is also referred to herein as the first cuff pressure signal. It should be noted that $P_{cuff}$ can also refer to an instantaneous measurement of the pressure in the first cuff 4 by the cuff pressure sensor 8 (where an instantaneous measurement is referred to herein as a cuff pressure measurement).

The volume oscillations generated inside the cuff 4 during the operation of the system 2 can be at a single frequency, (for example where arterial compliance is the arterial property to be determined), or at a plurality of different frequencies. In the latter case, cuff pressure measurements obtained when different frequencies of volume oscillations are generated in the cuff 4 can be used to obtain information about the viscosity of the artery. Alternatively or in addition, cuff pressure measurements obtained when one frequency of volume oscillations are generated in the cuff 4 can be used to estimate the compliance of the cuff 4, and cuff pressure measurements obtained when the other, or another, frequency of volume oscillations is used for determining information about the arterial compliance. The frequency(ies) of volume oscillations can be in the range of 1 Hz to 25 Hz. As described further below, the frequency of the volume oscillations should be different to the heart rate of the subject so that the effect of the volume oscillations generated in the cuff 4 can be observed in the cuff pressure measurements separately to oscillations due to blood flow in the artery. Typical heart rates are in the range of 0.8 Hz to 3Hz, and a suitable frequency of the volume oscillations can be above or below the heart rate.

In some embodiments, the system 2 comprises a second cuff 10.The second cuff 10 is to be placed around a body part of a subject of interest, in particular around the same limb as the first cuff 4 and upstream of the first cuff 4 (i.e. the second cuff 10 is closer to the heart of the subject than the first cuff 4; put another way, the second cuff 10 is proximal and the first cuff 4 is distal).The second cuff 10 can be connected to pump 6 via a connecting tube 11, and the pump 6 is controllable to selectively inflate the second cuff 10.The pump 6 may also be able to selectively deflate the second cuff 10, and/or a valve (not shown) may be provided for enabling the second cuff 10 to be deflated. It should be noted that the pump 6 inflates/deflates the second cuff 10 separately to the first cuff 4, e.g. the pump 6 can inflate or deflate the second cuff 10 to a particular pressure while the first cuff 4 is not inflated or deflated or is otherwise held at the current pressure, and vice versa. Furthermore, the pump 6 (or other oscillator component) is able to apply the volume oscillations to the first cuff 4 without affecting the pressure or volume in the second cuff 10 (i.e. without the volume oscillations being applied to the second cuff 10).In some embodiments, instead of using the first pump 6, a separate pump (not shown in Fig. 2) may be provided specifically for inflating/deflating the second cuff 10.

As described further below, during use of the system 2, the second cuff 10 can be inflated to a pressure that blocks the blood flow in the lower part of the limb (e.g. where the first cuff 4 is located), which means that blood does not flow in the artery beneath the first cuff 4.Volume oscillations can be generated in the first cuff 4 while the blood flow is blocked in the artery, and a cuff pressure signal obtained. Blocking the blood flow in this way enables the processing of the cuff pressure signal to be simplified when determining the information about the arterial property. Furthermore, blocking the blood flow in this way enables, in certain embodiments, a mean systemic filling pressure (MSFP) to be estimated for the subject from the cuff pressure signal.

The system 2 also comprises an apparatus 12 that operates according to the techniques described herein to determine information about one or more arterial properties for a subject. The apparatus 12 is configured to receive the cuff pressure signal from the cuff pressure sensor 8.In some embodiments, the apparatus 12 is configured to control the operation of the pump 6, and thereby initiate the inflation of the first cuff 4 at an appropriate time, and initiate the generation of the volume oscillations in the cuff 4.

The apparatus 12 may be in the form of, or be part of, a computing device, such as a server, desktop computer, laptop, tablet computer, smartphone, smartwatch, etc., or other types of device typically found in clinical environments, such as a patient monitoring device (e.g. a monitoring device located at the bedside of a patient in a clinical environment) that is used to monitor (and optionally display) various physiological characteristics of a subject/patient, including an arterial property such as arterial compliance and/or arterial viscosity.

The apparatus 12 includes a processing unit 22 that controls the operation of the apparatus 12 and that can be configured to execute or perform the methods described herein. The processing unit 22 can be implemented in numerous ways, with software and/or hardware, to perform the various functions described herein. The processing unit 22 may comprise one or more microprocessors or digital signal processors (DSPs) that may be programmed using software or computer program code to perform the required functions and/or to control components of the processing unit 22 to effect the required functions. The processing unit 22 may be implemented as a combination of dedicated hardware to perform some functions (e.g. amplifiers, pre-amplifiers, analog-to-digital convertors (ADCs) and/or digital-to-analog convertors (DACs)) and a processor (e.g., one or more programmed microprocessors, controllers, DSPs and associated circuitry) to perform other functions. Examples of components that may be employed in various embodiments of the present disclosure include, but are not limited to, conventional microprocessors, DSPs, application specific integrated circuits (ASICs), field-programmable gate arrays (FPGAs), hardware for implementing a neural network and/or so-called artificial intelligence (AI) hardware accelerators (i.e. a processor(s) or other hardware specifically designed for AI applications that can be used alongside a main processor).

The processing unit 22 is connected to a memory unit 24 that can store data, information and/or signals for use by the processing unit 22 in controlling the operation of the apparatus 12 and/or in executing or performing the methods described herein. In some implementations the memory unit 24 stores computer-readable code that can be executed by the processing unit 22 so that the processing unit 22 performs one or more functions, including the methods described herein. In particular embodiments, the program code can be in the form of an application for a smartwatch, smartphone, tablet, laptop or computer. The memory unit 24 can comprise any type of non-transitory machine-readable medium, such as cache or system memory including volatile and non-volatile computer memory such as random access memory (RAM), static RAM (SRAM), dynamic RAM (DRAM), read-only memory (ROM), programmable ROM (PROM), erasable PROM (EPROM) and electrically erasable PROM (EEPROM), and the memory unit 24 can be implemented in the form of a memory chip, an optical disk (such as a compact disc (CD), a digital versatile disc (DVD) or a Blu-Ray disc), a hard disk, a tape storage solution, or a solid state device, including a memory stick, a solid state drive (SSD), a memory card, etc.

In some embodiments, the apparatus 12 comprises a user interface 26 that includes one or more components that enables a user of apparatus 12 to input information, data and/or commands into the apparatus 12, and/or enables the apparatus 12 to output information or data to the user of the apparatus 12.Information that can be output by the user interface 26 can include any of: information about an arterial property, a value of an arterial property, information about arterial compliance, an arterial compliance value, information about viscosity of an artery, an arterial viscosity value, and a cuff compliance value. The user interface 26 can comprise any suitable input component(s), including but not limited to a keyboard, keypad, one or more buttons, switches or dials, a mouse, a track pad, a touchscreen, a stylus, a camera, a microphone, etc., and/or the user interface 26 can comprise any suitable output component(s), including but not limited to a display screen, one or more lights or light elements, one or more loudspeakers, a vibrating element, etc.

It will be appreciated that a practical implementation of an apparatus 12 may include additional components to those shown in Fig. 2.For example the apparatus 12 may also include a power supply, such as a battery, or components for enabling the apparatus 12 to be connected to a mains power supply. The apparatus 12 may also include interface circuitry for enabling a data connection to and/or data exchange with other devices, including servers, databases and user devices.

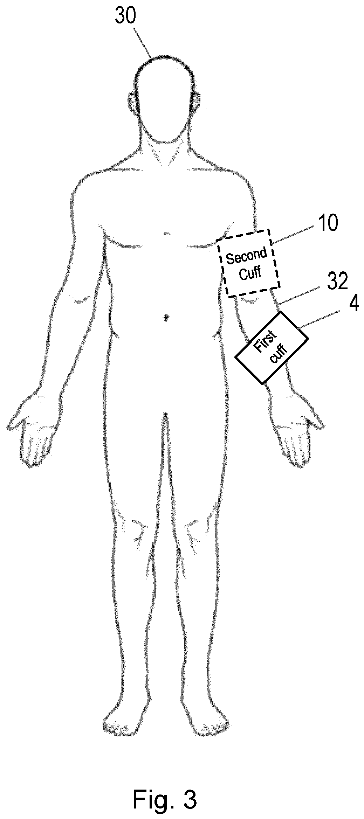

Fig. 3 illustrates an exemplary measurement setup with the first cuff 4, and optional second cuff 10, on a subject 30.The first cuff 4 is shown on the left arm 32 of the subject 30, and Fig. 3 also shows where the second cuff 10 can be placed in embodiments where the second cuff 10 is used. As noted above, the second cuff 10 should be around the same limb as the first cuff 4, and upstream of the first cuff 4.Thus, in this illustration, the second cuff 10 is on an upper part of the left arm 32, while the first cuff 4 is on a lower part of the left arm 32.This positioning of the cuffs 4, 10 allows the second cuff 10 to be inflated to a pressure that prevents blood flow into the lower part of the left arm 32 where the first cuff 4 is located. The pressure required in the second cuff 10 to prevent blood flow is above the systolic blood pressure of the subject 30.

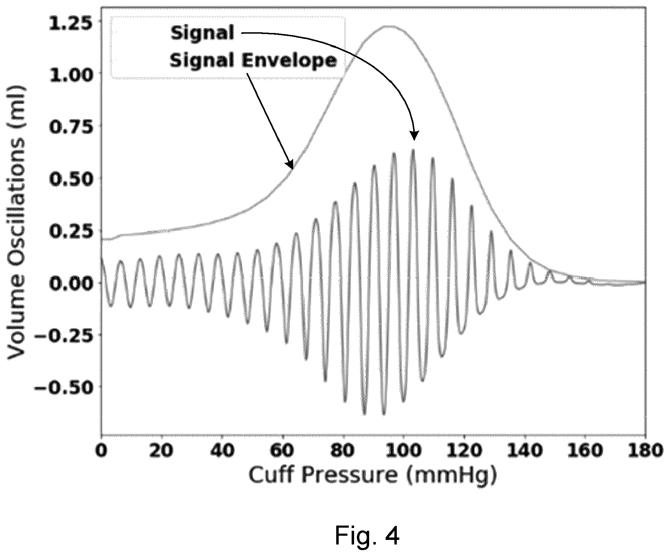

Fig. 4 is a graph illustrating the magnitude of arterial volume changes ($dV_{art}$) due to blood flow at different pressures in the cuff 4, in the absence of any artificial volume oscillations. Fig. 4 shows both the arterial volume oscillations (expressed in ml), and a signal envelope for the arterial volume oscillations (where the illustrated signal envelope is the difference between an upper envelope for the volume oscillations signal and a lower envelope for the volume oscillations signal (i.e. signal envelope = upper envelope - lower envelope)).

It has been found that measuring an arterial volume response to external pressure can be performed by generating well-defined volume oscillations inside the first cuff 4 placed on the arm 32 of a subject 30. Measuring the arterial volume response only requires a single cuff (the first cuff 4, although the first cuff 4 can be positioned on the upper or lower part of the arm 32). During use, the cuff 4 is inflated to different absolute pressures ($P_{abs}$), and volume oscillations ($dV_{osc}$) are generated artificially within the cuff 4 by the pump 6 while at various absolute pressures, and the pressure in the cuff 4 is measured by cuff pressure sensor 8. In some embodiments, a step-wise inflation/measurement approach can be used, in which, for example, the cuff 4 is inflated to a first absolute pressure, volume oscillations generated in the cuff 4 and the pressure in the cuff 4 measured while the volume oscillations are generated. The pressure in the cuff 4 is then changed to a second absolute pressure (which could be higher or lower than the first absolute pressure), volume oscillations generated in the cuff 4, the pressure in the cuff 4 measured, and so on. In alternative embodiments, a continuous inflation/measurement approach can be used, in which, for example, the cuff 4 is continuously inflated (e.g. at a constant rate of inflation) while volume oscillations are generated in the cuff 4, and the pressure in the cuff 4 is measured while the volume oscillations are generated. In either embodiment, the pressures in the cuff 4 can be in the range 0 to 200 mmHg.

If the compliance of the first cuff 4 ($C_{uff}$) is well-defined, then the pressure response $dP_{osc}$ of the cuff 4 to the artificial volume changes (the volume oscillations) is known from:

$$dP_{osc} = dV_{osc} / C_{cuff} \qquad (3)$$

The arterial volume $V_{art}$ responds to pressure oscillations $dP_{osc}$ and to internal pressure pulsations $dP_{art}$ (which occur between systolic and diastolic blood pressure values). The measured cuff pressure signal $P_{cuff}$ will therefore be influenced by both $dV_{art}$ and $dV_{osc}$. Arterial volume response can be can be obtained from a measurement of $P_{cuff}$ via the equation:

$$dV_{art} = \left(P_{cuff} - P_{abs}\right) * C_{cuff} - dV_{osc}( \qquad 4)$$

While equation (4) can be used to determine the arterial volume response for discrete measurements of the cuff pressure $P_{cuff}$, more generally, the arterial volume response can be derived using:

$$dV_{art}(t) = \left(P_{cuff}(t) - P_{abs}(t)\right) * C_{cuff} - dV_{osc}(t) \qquad (5)$$

where $dV_{art}(t)$ is the arterial volume response over time, and $P_{cuff}(t)$ is the cuff pressure signal.

The expected magnitude of the changes in arterial volume ($dV_{art}$) can be of the order of 0.25 ml at low cuff pressures and of the order of 1.25 ml as cuff pressure reaches mean arterial pressures, as shown in Fig. 4. In general, the maximum amplitude of $dV_{art}$ is expected to be in the range of 1 ml to 3 ml. This volume change occurs as a result of an arterial pressure change of the order of 40 mmHg (which is pulse pressure, or the difference between systolic and diastolic blood pressure values).

Since $C_{cuff}$ is not usually known prior to the inflation of the cuff 4, in some embodiments the measurement of pressure in the cuff 4 due to volume oscillations induced by the pump 6 can also be used to also determine cuff compliance from:

$$C_{cuff} = dV_{osc} / dP_{osc} \qquad (6)$$

Artificial volume oscillations of the order of 1 ml that cause a cuff pressure response of the order of 2 mmHg can be used to measure cuff compliance. The cuff pressure pulsations having a magnitude of the order of 2 mmHg do not cause the artery to oscillate. This is particularly so if the cuff compliance is measured at an absolute cuff pressure of 20 to 40 mmHg, or 140 to 180 mmHg (i.e. above the systolic blood pressure). At these pressures the arterial response to a pressure change of 2 mmHg would be of the order of 0.02 ml, which is negligible. This would effectively guarantee that the $dP_{osc}$ in response to $dV_{osc}$ is due to the cuff response, and in this amplitude range (1-2 mmHg artificial pressure pulsations), there is no interference with the artery.

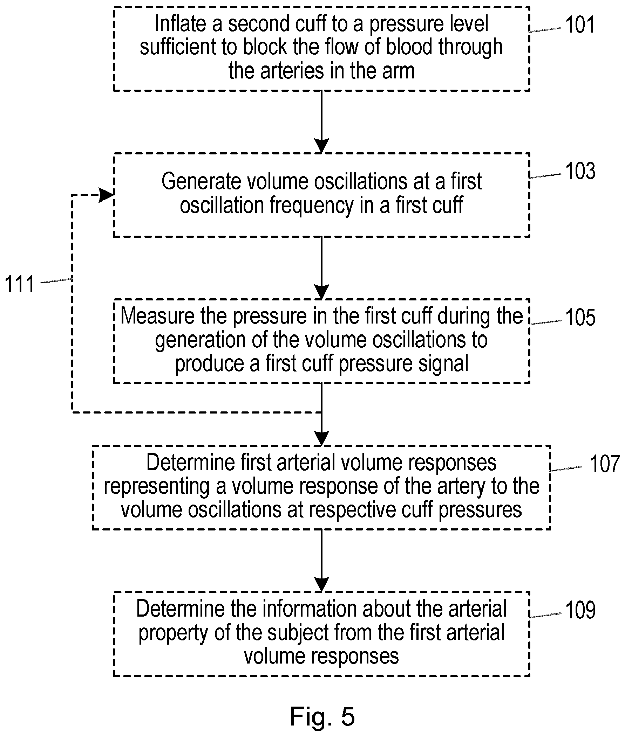

The flow chart in Fig. 5 illustrates an exemplary method of determining information about one or more arterial properties according to various embodiments. Various steps of this method can be performed using the processing unit 22, the first cuff 4, the pump 6 and the cuff pressure sensor 8. In particular, the processing unit 22 can control the operation of the pump 6 to inflate and deflate the first cuff 4. In some embodiments, the second cuff 10 can be present and inflated to block the blood flow in the artery/ies beneath the first cuff 4.

Briefly, in the method of Fig. 5, the volume response of the artery under cuff 4 is determined at a number of different pressures in the cuff 4, and information about one or more arterial properties is determined from the volume responses.

In a first step, step 101, which is optional, the second cuff 10 is placed on the arm 32 (or other limb) of the subject 30 and inflated (e.g. by the pump 6) to a pressure level that is sufficient to block the flow of blood through the arteries in the arm 32. This pressure level is referred to herein as a blocking cuff pressure, and is a pressure at or above the systolic blood pressure of the subject 30. In some embodiments, the systolic blood pressure of the subject 30 may be known, for example from having performed a blood pressure measurement using a conventional blood pressure measurement technique, but in other embodiments the blocking cuff pressure can be preset to a value that is above systolic blood pressures for a population of subjects.

In embodiments where the second cuff 10 is used, steps 103 and 105 are performed while the second cuff 10 is inflated to the blocking cuff pressure and blood flow in the arteries beneath the first cuff 4 is stopped. Otherwise, the second cuff 10 is not present or not used, and steps 103 and 105 are performed while blood is flowing through the arteries beneath the first cuff 4.

In step 103, volume oscillations are generated in the first cuff 4 at a first oscillation frequency. The first cuff 4 is at a first location on the arm 32 (or other limb) of the subject 30 (but the same limb as the second cuff 10 in embodiments where the second cuff 10 is used). The volume oscillations are generated in step 103 for a plurality of cuff pressures in the first cuff 4. The volume oscillations can be generated by the pump 6 or other oscillator component. The magnitude of the volume oscillations are well-defined, i.e. known. In some embodiments, the magnitude of the volume oscillations generated in the first cuff 4 are such that the resulting pressure oscillations in the cuff 4 are in the range of 20-50 mmHg. In some embodiments, the magnitude of the volume oscillations generated in the cuff 4 can be of the order of 10 ml, for example in the range of 5 ml to 20 ml. With a compliance of the first cuff 4 being 0.5 ml/mmHg, volume oscillations of magnitude 10 ml can cause pressure pulsations of the order of 20 mmHg.

In general, the first oscillation frequency can be a frequency in the range of 1 Hz to 25 Hz. In embodiments that do not use the second cuff 10, the first oscillation frequency should be different to the heart rate of the subject 30. For example, if the heart rate of the subject 30 is 60 beats per minute (bpm), i.e. 1 Hz, then the first oscillation frequency should be different to 1 Hz. As an example, with a heart rate of 60 bpm, the first oscillation frequency can be 3 Hz or higher. As another example, with a heart rate of 180 bpm (i.e. 3 Hz), the first oscillation frequency can be 1 Hz, or a frequency above 3 Hz.

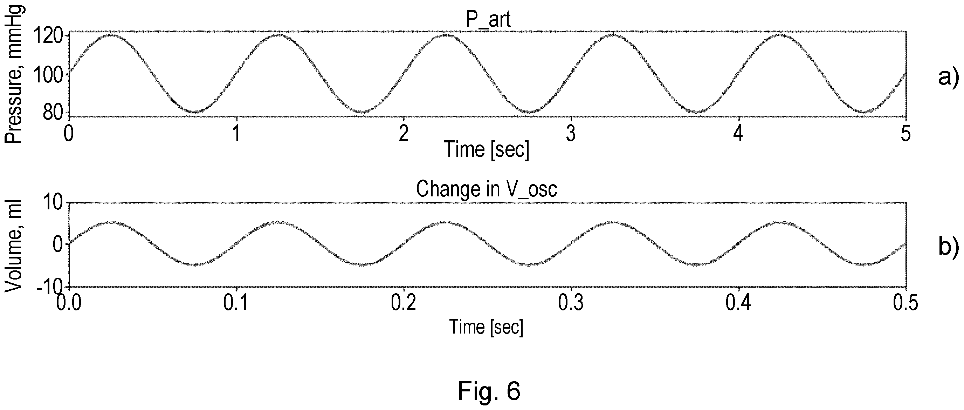

An example is provided with reference to Fig. 6. Fig. 6(a) is a graph illustrating arterial pressure ($P_{art}$) over time in the absence of any cuffs 4, 10 or volume oscillations. The arterial pressure $P_{art}$ represents blood pressure and shows changes between the systolic blood pressure (e.g. 120 mmHg) and the diastolic blood pressure (e.g. 80 mmHg) in accordance with the heart rate, which is 1 Hz in Fig. 6(a). Fig. 6(b) is a graph illustrating exemplary artificial volume oscillations (changes in $V_{osc}$) at a suitable oscillation frequency. Thus, the volume oscillations can have a magnitude of 20 ml (+10 ml to -10ml) and a frequency of 10 Hz.

In embodiments that use the second cuff 10 to block the flow of the blood in the arteries beneath the first cuff 4, the first oscillation frequency can be set to any desired value, but in the following it is assumed that volume oscillations according to Fig 6(b) are used.

In step 105, while the volume oscillations are being generated in the first cuff 4, the cuff pressure sensor 8 measures the pressure in the cuff 4. The cuff pressure sensor 8 produces a first cuff pressure signal that represents the pressure in the cuff 4 over time. The signal output by the cuff pressure sensor 8 is denoted $P_{cuff}$.

As noted above, a step-wise inflation/measurement approach can be used in which steps 103 and 105 comprise inflating the first cuff 4 to a first absolute pressure, generating volume oscillations in the cuff 4 and measuring the pressure in the cuff 4 while the volume oscillations are generated. The pressure in the cuff 4 is then changed to a second absolute pressure (which could be higher or lower than the first absolute pressure), volume oscillations generated in the cuff 4, the pressure in the cuff 4 measured, and so on. In alternative embodiments, a continuous inflation/measurement approach can be used in which steps 103 and 105 comprise continuously inflating the first cuff 4 (e.g. at a constant rate of inflation) while volume oscillations are continuously generated in the cuff 4, and the pressure in the cuff 4 is measured while the volume oscillations are generated. In either embodiment, the absolute pressures in the cuff 4 can be in the range 0 to 200 mmHg.

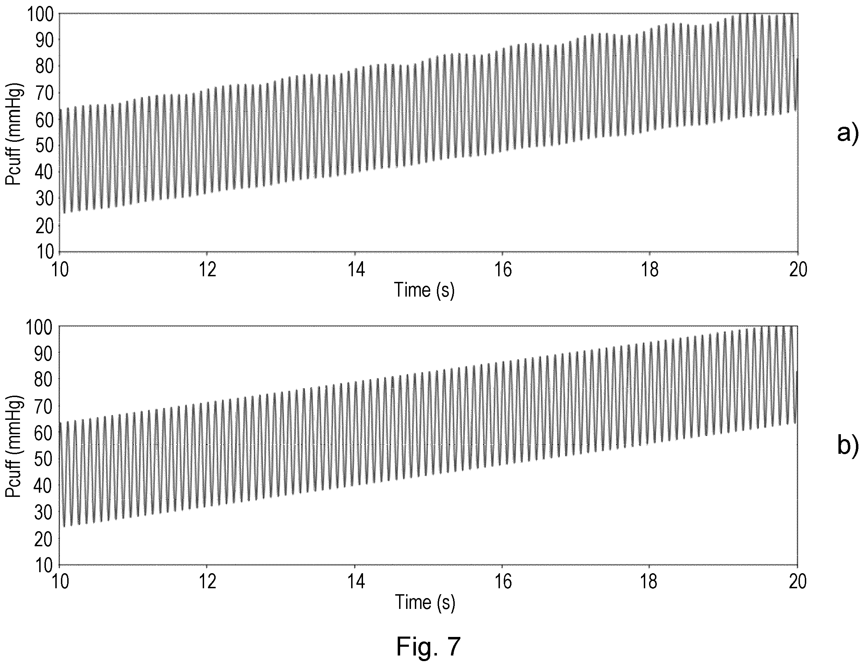

Fig. 7(a) illustrates a cuff pressure signal ($P_{cuff}$) obtained using the continuous inflation/measurement approach when blood is flowing through the artery beneath the first cuff 4. The cuff pressure signal is obtained by measuring the pressure in the first cuff 4 while the first cuff 4 is continuously inflated and the volume oscillations shown in Fig. 6(b) are generated. In this example the inflation rate of the first cuff 4 is around 3-4 ml/s, but it will be appreciated that other inflation rates can be used. At typical inflation rates for a cuff, a cuff can take between 10 s and 100 s to be inflated from 0 mmHg to above systolic blood pressure. The effect of the blood flow due to heart beats on the pressure in the first cuff 4 can be seen from the envelope of the cuff pressure signal in Fig. 7(a), since the higher frequency oscillations in the pressure in the first cuff 4 are caused by the artificial volume oscillations, and the lower frequency oscillations are caused by the blood flow.

Fig. 7(b) illustrates a cuff pressure signal ($P_{cuff}$) obtained using the continuous inflation/measurement approach when blood flow through the artery beneath the first cuff 4 is stopped through the use of the second cuff 10.As with Fig. 7(a), the cuff pressure signal in Fig. 7(b) is obtained by measuring the pressure in the first cuff 4 while the first cuff 4 is continuously inflated and the volume oscillations shown in Fig. 6(b) are generated. Again, in this example the inflation rate of the first cuff 4 is around 3-4 ml/s, but it will be appreciated that other inflation rates can be used. Again, at typical inflation rates for a cuff, a cuff can take between 10 s and 100 s to be inflated from 0 mmHg to above systolic blood pressure.

It will be appreciated that in the case of the step-wise inflation/measurement approach, there can be respective cuff pressure signals for each pressure 'step' at which cuff pressure measurements are made, in which case these signals are considered together in subsequent steps of the method, or there can be a single cuff pressure signal including the cuff pressure measurements at each of the pressure steps (in which case these pressure steps will be observable in the cuff pressure signal).

In step 107, the processing unit 22 determines arterial volume responses representing the volume response of the artery to the well-defined volume oscillations (at the first oscillation frequency) at different pressures in the first cuff 4.The arterial volume responses are determined from the cuff pressure signal obtained in step 105.In some embodiments, the arterial volume response is determined using the cuff compliance of the first cuff 4 ($C_{uff}$), and information about the volume oscillations generated in the first cuff 4 at the time of the cuff pressure measurement. The cuff compliance of the first cuff 4 ($C_{cuff}$) is either known, or has already been derived according to optional embodiments described further below. The information about the volume oscillations can be the maximum amplitude of the generated volume oscillations and the frequency of the volume oscillations. In some embodiments, the information about the volume oscillations can be a signal representing the change in volume generated in the first cuff 4 (this volume change signal is denoted $dV_{osc}$). The information about the volume oscillations can be, or be based on, a control signal used by the pump 6 or other oscillator component to generate the appropriate volume oscillations. In some embodiments, the arterial volume response is further determined using information indicating the absolute pressure in the first cuff 4 at the time that a particular cuff pressure measurement was obtained.

In some embodiments, the arterial volume response to the defined volume oscillations is determined using equation (4) or (5) above. Optionally, in these embodiments, ($P_{cuff}$ - $P_{abs}$) in equation (4) or (5) can be derived directly from the cuff pressure signal, for example by trend fitting of the cuff pressure signal.

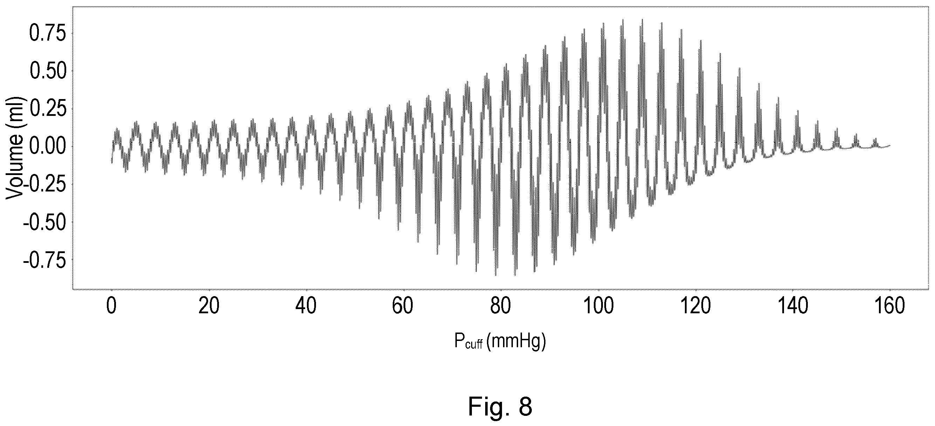

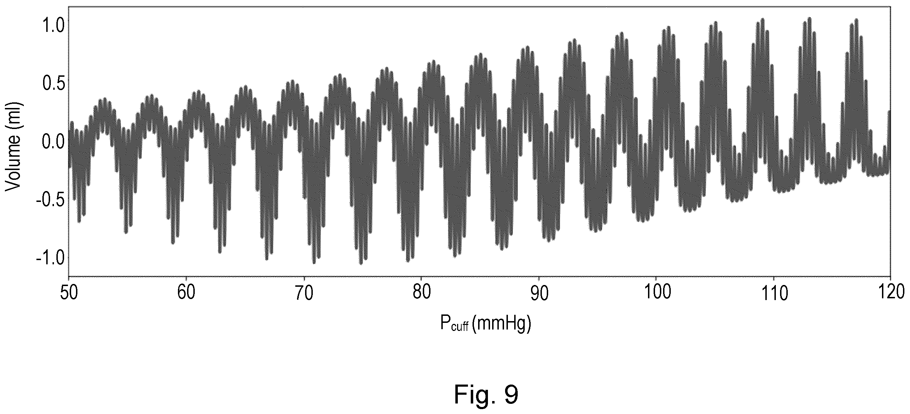

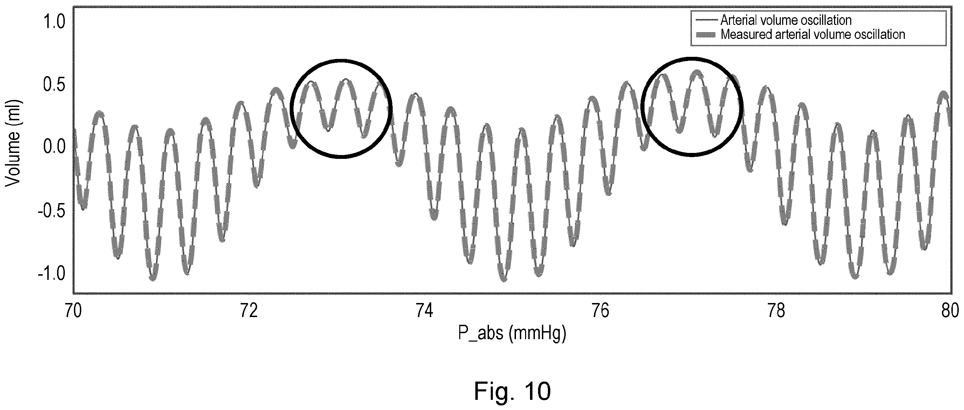

Fig. 8 illustrates an arterial volume response obtained from the cuff pressure signal in Fig. 7(a) using equation (4) or (5).In particular, Fig. 8 shows the volume response of the artery in ml (the y-axis) to the well-defined volume oscillations and to internal pressure oscillations at the first oscillation frequency at different pressures in mmHg (the x-axis) in the first cuff 4.Fig. 9 'zooms-in' on a part of the arterial volume response in Fig. 8.Fig. 10 further 'zooms-in' on a part of the arterial volume response in Fig. 9.

Preferably the arterial volume responses are determined from consistent parts of the cuff pressure signal. That is the arterial volume responses are determined from specific parts of the waveform in the cuff pressure signal. For example, as shown in Fig. 10, the arterial volume responses can be determined from the local maximas in the cuff pressure signal, i.e. where the cuff pressure signal represents the top of the arterial pressure pulse (where the arterial pressure is systolic blood pressure).Alternatively, the arterial volume responses can be determined from the local minimas in the cuff pressure signal, i.e. where the cuff pressure signal represents the bottom of the arterial pressure pulse (where the arterial pressure is diastolic blood pressure).

In embodiments where blood is flowing in the arteries beneath the first cuff 4 (i.e. in embodiments where the second cuff 10 is not used, or is not present in the system 2), step 107 can comprise band-pass filtering the first cuff pressure signal to obtain or extract signal components corresponding to the first oscillation frequency of the volume oscillations. That is, the first cuff pressure signal will include signal components (i.e. pressure changes) due to internally generated pulsations (i.e. the flow of blood at a frequency corresponding to the heart rate of the subject 30), and signal components (i.e. pressure changes) due to externally generated volume oscillations. Only the pressure changes due to the volume oscillations are of interest for determining the information about the arterial property, so the cuff pressure signal is band-pass filtered to extract the signal components caused by the volume oscillations. Thus, the band-pass filtering should pass (extract) a frequency band that includes, or is centered on, the first oscillation frequency, or, at the very least, pass a frequency band that does not include the heart rate of the subject 30.The passband of the band-pass filtering can be set based on the first oscillation frequency. The band-pass filtered cuff pressure signal includes the signal components corresponding to the first oscillation frequency of the volume oscillations and is used to determine the arterial volume response as described above. For example, ($P_{cuff}$ - $P_{abs}$) in equation (4) can be determined from the band-pass filtered cuff pressure signal, for example by performing a trend fitting of the cuff pressure signal to remove changes due to inflation, and a trend fitting of the resulting signal to remove internal pressure effects. As another example, further bandpass filtering can be used to determine $(P_{cuff} - P_{abs})$ in equation (4).

As an alternative to band-pass filtering the cuff pressure signal as described above, if the first oscillation frequency is much higher than the heart rate of the subject 30, then de-trending in the time domain can be used to extract the information about the response of the arteries to the external volume oscillations from the cuff pressure signal. For example a slow-changing trend can be fitted to the cuff pressure signal shown in Fig. 7(a), and the signal of interest can be obtained as the difference between the signal in Fig. 7(a) and the trend.

Finally, in step 109, information about the arterial property of the subject 30 is determined from the arterial volume responses determined in step 107. The arterial property can be arterial compliance and/or viscosity of the artery.

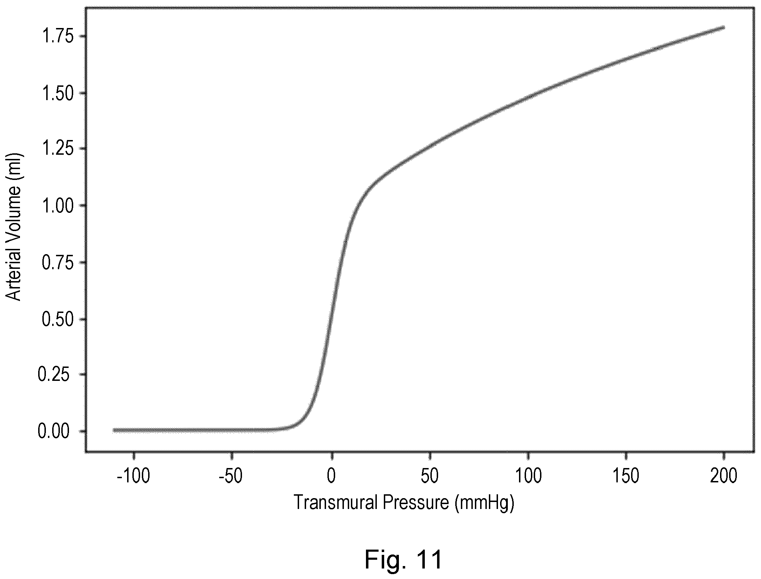

Information about an arterial property, for example arterial compliance or viscosity of the artery, can be determined from a function of arterial volume response against transmural pressure (i.e. the pressure applied across the arterial wall), noting that the transmural pressure $(P_{tm})$ decreases as the cuff is inflated (from a low pressure, e.g. 0 mmHg). The paper "Theory of the Oscillometric Maximum and the Systolic and Diastolic Detection Ratios" by G. Drzewiecki et al., Annals of Biomedical Engineering, Vol. 22, pp. 88-96, 1994 describes how the transmural pressure is related to the behavior of arterial collapse. The function of arterial volume response against transmural pressure is determined from the arterial volume response with respect to the defined pressure pulsation determined in step 107. Exemplary techniques for determining the function of arterial volume response against transmural pressure from the arterial volume response with respect to the defined pressure oscillations are described in WO 2020/126576 ("Control unit for deriving a measure of arterial compliance"), and the paper "A modelling framework for assessment of arterial compliance by fusion of oscillometry and pulse wave velocity information" by Bogatu, L. I., Turco, S., Mischi, M., Woerlee, P., Bouwman, A., Korsten, E. H. H. M., & Muehlsteff, J., Computer Methods and Programs in Biomedicine, 2020, 196. Fig. 11 is a graph illustrating an arterial volume response (in ml) as a function of transmural pressure (in mmHg). Based on equation (1), it can be seen that the relationship illustrated in Fig. 11 represents the arterial compliance as a function of transmural pressure, as thus the function of arterial volume response against transmural pressure provides the information on the arterial compliance of the subject 30.

In embodiments where the arterial property is viscosity of the artery, the viscosity information can be obtained based on the response of the artery to volume oscillations at a plurality of different frequencies. In that case, if information about viscosity is desired, then steps 103 and 105 are repeated for one or more further oscillation frequencies for the volume oscillations, as indicated by arrow 111. The one or more further oscillation frequencies are different to the first oscillation frequency and can be any frequency in the range of 1 Hz to 25 Hz. As with the first oscillation frequency, in embodiments that do not use the second cuff 10, the further oscillation frequency(ies) should be different to the heart rate of the subject 30.

Repetition of steps 103 and 105 provides a respective further cuff pressure signal for each further oscillation frequency. In some embodiments (which are primarily applicable to the continuous inflation/measurement approach), once steps 103 and 105 are performed for the first oscillation frequency, the first cuff 4 can be deflated to an initial pressure level, and steps 103 and 105 performed at a second oscillation frequency while the first cuff 4 is inflated again. This provides a cuff pressure signal for the second oscillation frequency. This can be repeated for each further oscillation frequency. In alternative embodiments (which are primarily applicable to the step-wise inflation/measurement approach), once a cuff pressure signal has been obtained for the first oscillation frequency at a particular pressure 'step', volume oscillations at a further oscillation frequency can be applied and a further cuff pressure signal obtained. This can be repeated for each further oscillation frequency, and at each pressure 'step'.

In an alternative to repeating steps 103 and 105 to provide respective further cuff pressure signals for each further oscillation frequency, rather than the volume oscillations applied in step 103 having a single frequency, volume oscillations of multiple frequencies can be combined and the multiple-frequency volume oscillations applied in step 103. For example a 3 Hz sine wave and a 10 Hz sine wave can be combined, and this combined signal used to drive the volume oscillations. It will be appreciated that this embodiment requires the resulting cuff pressure signal to be processed to separate the arterial volume responses due to different oscillation frequencies. This can be achieved through the use of band-pass filters to extract the signal components due to a respective oscillation frequency.

Whichever approach is used to obtain the cuff pressure signal for each further oscillation frequency, the resulting cuff pressure signals are processed according to step 107 to determine further arterial volume responses at each of the oscillation frequencies. The further arterial volume responses for a particular further oscillation frequency are determined based on the respective further cuff pressure signal, the cuff compliance of the first cuff, and a respective volume oscillation signal related to the particular further oscillation frequency of the volume oscillations, similar to step 107 above. As with step 107 above, the arterial volume responses can be determined according to equations (4) or (5).

It will be appreciated that it is possible for the repetition of steps 103 and 105 to be performed before any iteration of step 107 is performed (e.g. prior to determining the arterial volume responses from the cuff pressure signal obtained for the first oscillation frequency).

In step 109, the information about the arterial property of the subject 30 (e.g. the viscosity of the artery) can be determined from the first arterial volume responses and the further arterial volume responses. In particular, viscosity information can be derived from the different arterial volume responses to external volume oscillations at different frequencies.

As an example, information about the viscosity can be provided as a comparison of the arterial volume responses to volume oscillations at one oscillation frequency to the arterial volume responses to volume oscillations at one or more other oscillation frequencies. For example, the arterial volume response to 10 Hz volume oscillations and can be compared to the arterial volume responses to 1Hz volume oscillations. Changes in viscosity over time (e.g. due to degradation or improvement of the subject's health) can be observed by comparing arterial volume responses obtained in different measurement operations. The information about viscosity of the artery can be stored in the form of a look-up table, with each table entry indicating, for example, the arterial volume response at a certain transmural pressure and certain oscillation frequency. Other information about the viscosity can be provided by the arterial volume responses to volume oscillations with low frequencies (which can be considered to describe the viscosity behavior at slow changes of stress), and arterial volume responses to volume oscillations at higher frequencies (which can be considered to indicate how the arteries respond to periods of high load (i.e. higher heart rate).

As noted, the information about the arterial property can be presented in a number of ways. For example the information about the arterial compliance can be expressed in the form of $dV_{art}(t)/dP_{ext}(t)$ (e.g. the relationship shown in Fig. 11)

or $|max(dV_{art})|/|max(dP_{ext})|$. As another example, the information about the viscosity of the artery can be expressed in the form of:

$$dV_{art}(1\text{Hz, time1})/dV_{art}(10\text{Hz, time1}) - dV_{art}(1\text{Hz, time2})/dV_{art}(10\text{Hz, time2}) \qquad (7)$$

or simply $dV_{art}(10\text{Hz})$ - $dV_{art}(1\text{Hz})$ at a respective transmural pressure. The expressions all contain information on the viscosity of the artery. The arterial volume responses and/or function of arterial volume response against transmural pressure can also be input to models describing the artery via elasticity/viscosity parameters. For example, measurements at different frequencies/transmural pressures can be fitted to an artery model to infer elasticity/viscosity parameters.

As noted above, the arterial volume responses are determined based on the compliance of the first cuff 4. In some embodiments, the cuff compliance $C_{cuff}$ is known or derived before the method in Fig. 5 is performed. Techniques for deriving the compliance of a cuff are known in the art, for example in EP 3705033 ("Blood pressure measurement device and control method") and the paper "Insights into oscillometry: An Experimental Study for Improvement of Cuff-Based Blood Pressure Measurement Technology" by Bogatu, L., Muehlsteff, J., Bresch, E., Smink, J., & Woerlee, P. H.., 2019 41st Annual International Conference of the IEEE Engineering in Medicine and Biology Society, EMBC 2019 (pp. 7068-7071).

In alternative embodiments, the compliance of the first cuff 4 can be determined using the system 2 before, or as part of, the method in Fig. 5. This can be useful as it enables the cuff compliance to be determined at the start of the arterial property measurement technique, and could allow, for example, the use of different cuffs with the system 2 (e.g. different sized cuffs for different sized subjects). In any case, the cuff compliance is required for determining the arterial volume responses in step 107, and so the cuff compliance should be determined or available prior to those steps being performed (e.g. before step 101 (where applicable), before steps 103 and 105, or after steps 103 and 105). The cuff compliance can be determined in embodiments where the second cuff 10 is also present and blocking the flow of blood in the artery, or in embodiments where the second cuff 10 is omitted or not used (or only used when obtaining the information about the arterial property).

To determine the cuff compliance, the first cuff 4 is initially inflated to a particular pressure level (also referred to herein as a third cuff pressure). Any particular pressure level can be used, and in some embodiments the particular pressure level is 80 mmHg.

While the pressure in the first cuff 4 is at the particular pressure level, volume oscillations are generated in the first cuff 4 by the pump 6 or other oscillator component. These volume oscillations typically have a smaller, or much smaller, magnitude than the volume oscillations generated (or to be generated) in step 103. In some embodiments, the magnitude of the volume oscillations generated in the first cuff 4 can be 1 ml, or of the order of 1 ml, and the frequency of these volume oscillations can be 8 Hz, or around 8 Hz. The volume oscillations generated here aim to induce pressure changes of the order of 2 mmHg in the cuff 4., i.e. much lower than the pressure changes caused by the volume oscillations generated in step 103. The cuff pressure sensor 8 measures the pressure in the first cuff 4 as the volume oscillations are generated.

The cuff pressure signal is then band-pass filtered to extract the pressure response in the first cuff 4 due to the volume oscillations. As in the above embodiments of step 107, the aim is to filter out (i.e. remove) the pressure response due to the blood flow in the artery, which has a frequency of the order of 1 Hz (60 BPM). Thus, the cuff pressure signal is band-pass filtered with the pass band being centered on or including the frequency of the volume oscillations (e.g. 8 Hz) to extract the pressure response due to the volume oscillations. The band pass filtering of the second cuff pressure signal results in a filtered cuff pressure signal.

The cuff compliance of the first cuff 4 can then be determined from the filtered cuff pressure signal. In particular, based on equation (3) above, the change in volume in the first cuff 4 due to the volume pulsations (i.e. the amplitude of the volume oscillations, e.g. 1 ml), $dV_{osc}$, is divided by the amplitude of the filtered cuff pressure signal ($dP_{osc}$) to give the cuff compliance, $C_{cuff}$. As noted above, further details of the derivation of cuff compliance can be found in, for example, EP 3705033 and/or the paper "Insights into oscillometry: An Experimental Study for Improvement of Cuff-Based Blood Pressure Measurement Technology". This cuff compliance can then be used in step 107 to determine the arterial volume responses.

In addition to determining information about an arterial property such as arterial compliance or viscosity of an artery, the assessment of fluid responsiveness by means of calculating mean systemic filling pressure (MSFP) is another application which can benefit from quantification of arterial compliance. As noted below, MSFP can be derived by measurement of stop-flow arterial and venous equilibrium pressures. These pressure values can be input to a Guytonian model, to infer the mean systemic filling pressure, and further, the effective intravascular volume status, which contributes to the assessment of fluid responsiveness, as described in "Estimation of mean systemic filling pressure in postoperative cardiac surgery patients with three methods" by Maas JJ et al., Intensive Care Med. 2012;38:1452-1460 and "Transient stop-flow arm arterial-venous equilibrium pressure measurement: determination of precision of the technique" by Aya HD et al., J Clin Monit Comput. 2016;30(1):55-61.Information about arterial compliance can improve the accuracy of the model parameters, thus leading to a better quantification of fluid responsiveness.

The MSFP is defined as the mean pressure that exists in the circulatory system when there is no blood in motion.MSFP is influenced by the volume of blood and the smooth muscle tone.MSFP can be derived by measurement of stop-flow arterial and venous equilibrium pressures. Occluding the vein and the artery at a particular site (e.g. a brachial site) causes the flow of blood at the distal part of the limb (e.g. lower arm) to stop. Conventionally MSFP is determined by measuring blood pressure at the distal limb site during occlusion.

However, MSFP can be obtained using system 2 in Fig 2 without having to measure blood pressure. To determine the MSFP, firstly information on the arterial compliance of the subject 30 is determined according to the method in Fig. 5, with the second cuff 10 inflated to the blocking cuff pressure so that the blood flow in the artery beneath the first cuff 4 is stopped. The information about the arterial compliance can be in the form of the arterial volume vs transmural pressure function shown in Fig. 11.

From the function of arterial volume response against transmural pressure, the cuff pressure in the first cuff 4 at which the maximum arterial compliance occurs is determined. Arterial compliance is given by equation (1), and thus the arterial compliance at a particular transmural pressure corresponds to the gradient of the function shown in Fig. 11 at that transmural pressure. Thus, it can be seen from Fig. 11 that the maximum arterial compliance occurs at a transmural pressure of 0 mmHg. As there is no blood flow beneath the first cuff 4 during the measurement of arterial compliance, the cuff pressure required to achieve this transmural pressure of 0 mmHg is equal to the MSFP. Thus, the MSFP for the subject 30 is determined as the cuff pressure in the first cuff 4 at which the maximum arterial compliance occurs. Thus, this embodiment provides for a non-invasive measurement of MSFP.

In a further approach, information about arterial compliance could also be used in the assessment in limb blood flow, measured by means of venous occlusion plethysmography. This measurement is also based on employing a model which describes the physiology of the occluded limb, as described in "Interpretation of venous occlusion plethysmographic measurements using simple model" by Seagar, A.D. et al., Med. Biol. Eng. Comput. (1984) 22: 12.Accurate estimation of the model's parameters (e.g. arterial compliance) can help interpret changes in limb volume during this measurement (e.g. detection of venous thrombosis).

Further details of step 107 and 109 above are provided below. A simulation framework is established to show how a cuff pressure signal is generated in the presence of artificial oscillations and the artery response to the artificial oscillations (forward model), and to show how the cuff pressure signal can be processed to obtain the response of the artery (an inverse model).The simulation framework is characterized by the following equations:

$$P_{cuff} = P_{abs} + (V_{osc} + V_{art})/C_{cuff} \qquad (8)$$

$$V_{art}(P_{tm})$$
$$= 0.08 * \frac{\log(0.03 * P_{tm} + 3.3)}{1 + \exp(-0.1 * P_{tm})} * 14 \qquad (9)$$

In equation (9), $V_{art}$ is the arterial volume under the first cuff 4 as a function of transmural pressure across the

arterial wall (denoted $P_{tm}$).Equation (9) is found in the paper "Theory of the Oscillometric Maximum and the Systolic and Diastolic Detection Ratios" by G. Drzewiecki et al. referenced above.

Based on equation (2), with the transmural pressure $P_{tm}$ being provided by the first cuff 4:

$$P_{tm} = P_{art} - P_{cuff} \qquad\qquad (10)$$

$P_{art}$ is the arterial pressure which oscillates between systolic blood pressure and diastolic blood pressure.

The first cuff 4 is simulated to inflate from 0 to 200 mmHg (i.e. $P_{abs}$ gradually increases).$P_{art}$ oscillates between 80 mmHg and 120 mmHg at roughly a frequency of 1 Hz due to the flow of blood in the artery, as shown by the graph in Fig 6(a).In this example $V_{osc}$ oscillates at 10 Hz, with a 10 ml amplitude, as shown in Fig. 6(b).In this simulation, the cuff compliance is set to 0.5 ml/mmHg.

As $P_{abs}$ increases, $P_{art}$ and $V_{osc}$ oscillate, and the pressure inside the cuff 4 is measured. This generates the cuff pressure signal shown in Fig. 7(a).Fig. 7(a) shows arterial volume oscillations occurring due to resulting transmural pressure $P_{tm}$ pulsations. When compared to the signal illustrated in Fig. 4 (which is caused by internal arterial oscillations), the arterial volume pulsations in Fig. 7(a) are caused by a combination of internal arterial pressure and external cuff pressure pulsations. Figs. 8 and 9 show the de-trended or band-pass filtered pressure signal of Fig. 7(a).

During an actual measurement of cuff pressure, the cuff pressure signal in Fig. 8 would be recorded and the oscillation volume ($dV_{art}$) would be known. The arterial volume pulsations can be obtained using equation (4) or (6), the recorded cuff pressure signal and the known volume oscillation amplitude. Fig. 10 shows the reconstructed arterial volume oscillations plotted together with the original arterial volume signal.

As noted above, the arterial compliance and other arterial collapse characteristics can be obtained by reconstructing the arterial volume response vs transmural pressure function, as illustrated in Fig. 11.As noted, this function can be achieved by obtaining the arterial volume responses to artificial pressure oscillations of amplitude 20 mmHg. For example, in Fig. 10 (a 'zoom in' of part of Fig. 9), the larger amplitude low frequency pulses are generated by internal arterial pressure, i.e. due to blood flow. The fast, smaller volume oscillations are the ones occurring due to the 20 mmHg artificial pressure pulsations. The arterial volume response $dV_{art}$ in response to 20mmHg pressure changes is obtained at several cuff pressures. To minimize the impact of arterial internal pressure oscillations, it is useful to process a specific portion of the waveform, as illustrated in Fig. 10.In Fig. 10, the top (peak) of the arterial pressure pulse, where the arterial pressure is always the systolic value, can be the part of the waveform that is processed to determine the arterial compliance. In alternative embodiments, the troughs of the arterial pressure pulse, where the arterial pressure is always the diastolic value, can be the part of the waveform that is processed to determine the arterial compliance. The arterial compliance values at different cuff pressures can be determined parametrically or non-parametrically to obtain the arterial volume vs transmural pressure function. Exemplary techniques for determining the function of arterial volume response against transmural pressure are described in WO 2020/126576, entitled "Control unit for deriving a measure of arterial compliance", and the paper "A modelling framework for assessment of arterial compliance by fusion of oscillometry and pulse wave velocity information".

Therefore the above-described techniques provide improved ways to determine information about arterial properties such as arterial compliance and viscosity of the artery non-invasively. These techniques do not require the measurement of blood pressure, which can be a source of measurement errors in conventional techniques. In addition, the described techniques can be performed using existing equipment or apparatus that is used for patient monitoring.

Variations to the disclosed embodiments can be understood and effected by those skilled in the art in practicing the principles and techniques described herein, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. A computer program may be stored or distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

**Claims**

1. A method for determining information about an arterial property of an artery of a subject, the method comprising:

generating (103) volume oscillations at a first oscillation frequency in a first cuff that is at a first location on a limb of the subject, wherein the volume oscillations are generated at a plurality of cuff pressures in the first cuff;

measuring (105) the pressure in the first cuff during the generation of the volume oscillations to produce a first cuff pressure signal;

determining (107) first arterial volume responses representing a volume response of the artery to the volume oscillations at respective cuff pressures, wherein the first arterial volume responses are determined based on the first cuff pressure signal, a cuff compliance of the first cuff, and a volume oscillation signal related to the first oscillation frequency of the volume oscillations generated in the first cuff; and

determining (109) the information about the arterial property of the subject from the first arterial volume responses.

2. A method as claimed in claim 1, wherein step (c) further comprises:

band-pass filtering the first cuff pressure signal to obtain signal components corresponding to the first oscillation frequency of the volume oscillations; and

determining the first arterial volume response based on the band-pass filtered first cuff pressure signal.

3. A method as claimed in claim 1, wherein the method further comprises:

prior to step (a), inflating (101) a second cuff that is at a second location on the subject to a blocking cuff pressure that is a cuff pressure above the systolic blood pressure of the subject, wherein the second location is on the same limb as, and upstream of, the first location; and

wherein steps (a) and (b) are performed while the second cuff is inflated to the blocking cuff pressure.

4. A method as claimed in claim 3, wherein step (d) comprises:

determining a function of arterial volume response against transmural pressure from the first arterial volume response; and

determining the information about the arterial property from the determined function.

5. A method as claimed in any of claims 1-4, wherein the arterial property comprises arterial compliance, and the method further comprises:

determining the cuff pressure in the first cuff at which a maximum arterial compliance occurs from the function of arterial volume response against transmural pressure; and

determining a mean systemic filling pressure, MSFP, for the subject as the cuff pressure in the first cuff at which the maximum arterial compliance occurs.

6. A method as claimed in any of claims 1-5, wherein the method further comprises:

repeating steps (a) and (b) for one or more further oscillation frequencies for the volume oscillations to produce a respective one or more further cuff pressure signals;

determining further arterial volume responses for each of the one or more further oscillation frequencies, the further arterial volume responses representing a volume response of the artery to the volume oscillations at respective cuff pressures, wherein an arterial volume response is determined based on the respective further cuff pressure signal, the cuff compliance of the first cuff, and a respective volume oscillation signal related to the respective oscillation frequency of the volume oscillations generated in the first cuff; and

wherein step (d) comprises determining the information about the arterial property of the subject from the first arterial volume responses and the further arterial volume responses.

7. A method as claimed in claim 6, wherein the arterial property is a viscosity of the artery, wherein step (d) comprises determining the information about the viscosity of the artery from a comparison of the first arterial volume responses and the further arterial volume responses.

8. A method as claimed in any of claims 1-7, wherein the method further comprises determining the cuff compliance of the first cuff by, prior to step (c):

generating volume oscillations at a second oscillation frequency in the first cuff when the first cuff is inflated to a third cuff pressure;

measuring the pressure in the first cuff during the generation of the volume oscillations at the second oscillation frequency to produce a third cuff pressure signal;
band-pass filtering the third cuff pressure signal to obtain signal components corresponding to an oscillation frequency of the second volume oscillations; and
determining the cuff compliance of the first cuff from the filtered third cuff pressure signal.

9. A computer program product comprising a computer readable medium having computer readable code embodied therein, the computer readable code being configured such that, on execution by a suitable computer or processing unit, the computer or processing unit is caused to perform the method of any of claims 1-8.

10. An apparatus (12) for determining information about an arterial property of an artery of a subject (30), the apparatus (12) configured to:

initiate generation of volume oscillations at a first oscillation frequency in a first cuff (4) that is at a first location on a limb of the subject (30), wherein the volume oscillations are generated at a plurality of cuff pressures in the first cuff (4);
obtain a first cuff pressure signal that comprises measurements of the pressure in the first cuff (4) during the generation of the volume oscillations;
determine first arterial volume responses representing a volume response of the artery to the volume oscillations at respective cuff pressures, wherein the first arterial volume responses are determined based on the first cuff pressure signal, a cuff compliance of the first cuff (4), and a volume oscillation signal related to the first oscillation frequency of the volume oscillations generated in the first cuff (4); and
determine the information about the arterial property of the subject (30) from the first arterial volume responses.

11. An apparatus (12) as claimed in claim 10, wherein the apparatus (12) is further configured to:

prior to operation (a), initiate inflation of a second cuff (10) that is at a second location on the subject (30) to a blocking cuff pressure that is a cuff pressure above the systolic blood pressure of the subject (30), wherein the second location is on the same limb as, and upstream of, the first location; and
wherein operations (a) and (b) are performed while the second cuff (10) is inflated to the blocking cuff pressure.

12. An apparatus (12) as claimed in claim 11, wherein operation (d) comprises:

determining a function of arterial volume response against transmural pressure from the first arterial volume response; and
determining the information about the arterial property from the determined function.

13. An apparatus (12) as claimed in any of claims 10-12, wherein the arterial property comprises arterial compliance, and the apparatus (12) is further configured to:

determine the cuff pressure in the first cuff (4) at which a maximum arterial compliance occurs from the function of arterial volume response against transmural pressure; and
determine a mean systemic filling pressure, MSFP, for the subject as the cuff pressure in the first cuff (4) at which the maximum arterial compliance occurs.

14. An apparatus (12) as claimed in any of claims 10-13, wherein the apparatus (12) is further configured to:

repeating operations (a) and (b) for one or more further oscillation frequencies for the volume oscillations to produce a respective one or more further cuff pressure signals;
determine further arterial volume responses for each of the one or more further oscillation frequencies, the further arterial volume responses representing a volume response of the artery to the volume oscillations at respective cuff pressures, wherein an arterial volume response is determined based on the respective further cuff pressure signal, the cuff compliance of the first cuff (4), and a respective volume oscillation signal related to the respective oscillation frequency of the volume oscillations generated in the first cuff (4); and
wherein operation (d) comprises determining the information about the arterial property of the subject (30) from the first arterial volume responses and the further arterial volume responses.

15. An apparatus (12) as claimed in claim 14, wherein the arterial property is a viscosity of the artery, wherein operation (d) comprises determining the information about the viscosity of the artery from a comparison of the first arterial volume responses and the further arterial volume responses.

Fig. 1

Fig. 2

EP 4 011 284 A1

Fig. 3

Fig. 4

Fig. 5

Fig. 6

Fig. 7

Fig. 8

EP 4 011 284 A1

Fig. 9

Fig. 10

EP 4 011 284 A1

Fig. 11

Europäisches Patentamt

European Patent Office

Office européen des brevets

**EUROPEAN SEARCH REPORT**

Application Number

EP 20 21 3169

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2014/135634 A1 (PRANEVICIUS OSVALDAS [US] ET AL) 15 May 2014 (2014-05-15) * paragraphs [0059], [0092], [0093], [0100] - [0106], [0135] - [0141], [0156] - [0159], [0174] - [0186] * | 1-15 | INV. A61B5/02 A61B5/022 ADD. A61B5/021 A61B5/00 |
| X | US 2011/263990 A1 (FLAHERTY BRYAN PATRICK [US] ET AL) 27 October 2011 (2011-10-27) * paragraphs [0010] - [0013], [0028], [0086] - [0095] * | 1,2,9,10 | |
| X | EP 3 705 033 A1 (KONINKLIJKE PHILIPS NV [NL]) 9 September 2020 (2020-09-09) * paragraphs [0013] - [0034], [0067] - [0069], [0080] - [0083], [0090], [0100], [0110] - [0114], [0137] - [0143] * | 1,3-5, 9-13 | |

TECHNICAL FIELDS SEARCHED (IPC)

A61B

The present search report has been drawn up for all claims

| Place of search | Date of completion of the search | Examiner |
|---|---|---|
| The Hague | 31 May 2021 | Kowalczyk, Szczepan |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 20 21 3169

This annex lists the patent family members relating to the patent documents cited in the above-mentioned European search report.
The members are as contained in the European Patent Office EDP file on
The European Patent Office is in no way liable for these particulars which are merely given for the purpose of information.

31-05-2021

| Patent document cited in search report | | Publication date | Patent family member(s) | | Publication date |
|---|---|---|---|---|---|
| US 2014135634 | A1 | 15-05-2014 | NONE | | |
| US 2011263990 | A1 | 27-10-2011 | CA | 2258263 A1 | 31-12-1997 |
| | | | EP | 0955868 A1 | 17-11-1999 |
| | | | EP | 1743572 A1 | 17-01-2007 |
| | | | JP | 3957758 B2 | 15-08-2007 |
| | | | JP | 2000512875 A | 03-10-2000 |
| | | | US | 6027452 A | 22-02-2000 |
| | | | US | 2002099296 A1 | 25-07-2002 |
| | | | US | 2004077956 A1 | 22-04-2004 |
| | | | US | 2006004293 A1 | 05-01-2006 |
| | | | US | 2006206030 A1 | 14-09-2006 |
| | | | US | 2010056930 A1 | 04-03-2010 |
| | | | US | 2011263990 A1 | 27-10-2011 |
| | | | WO | 9749328 A1 | 31-12-1997 |
| EP 3705033 | A1 | 09-09-2020 | NONE | | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2020126576 A

- EP 3705033 A

### Non-patent literature cited in the description

- **FERNANDO PABLO SALVUCCI et al.** Arterial wall mechanics as a function of heart rate: role of vascular smooth muscle. *J. Phys.: Conf. Ser. 90 012010,* 2007
- Beat to Beat Modulation of Arterial Pulse Wave Velocity Induced by Vascular Smooth Muscle Tone. **L. J. CYMBERKNOP et al.** 41st Annual International Conference of the IEEE Engineering in Medicine and Biology Society. EMBC, 2019, 5030-5033
- **WILKINSON IB ; WEBB DJ, BR.** Venous occlusion plethysmography in cardiovascular research: methodology and clinical applications. *J. Clin. Pharmacol.,* 2001, vol. 52, 631-646
- **RAS, R. T. et al.** Flow-mediated dilation and cardiovascular risk prediction: a systematic review with meta-analysis. *Int J Cardiol,* vol. 168, 344-351
- **SELVARAJ N et al.** Influence of respiratory rate on the variability of blood volume pulse characteristics. *J Med Eng Technol.,* 2009, vol. 33 (5), 370-375
- **WU HT et al.** Assessment of Vascular Health With Photoplethysmographic Waveforms From the Fingertip. *IEEE J Biomed Health Inform.,* March 2017, vol. 21 (2), 382-386
- **ZAHEDI E.** Finger photoplethysmogram pulse amplitude changes induced by flow-mediated dilation. *Physiol Meas.,* 2008, vol. 29, 625-37
- **G. DRZEWIECKI et al.** Theory of the Oscillometric Maximum and the Systolic and Diastolic Detection Ratios. *Annals of Biomedical Engineering,* 1994, vol. 22, 88-96

- **BOGATU, L. I. ; TURCO, S. ; MISCHI, M. ; WOERLEE, P. ; BOUWMAN, A. ; KORSTEN, E. H. H. M. ; MUEHLSTEFF, J.** A modelling framework for assessment of arterial compliance by fusion of oscillometry and pulse wave velocity information. *Computer Methods and Programs in Biomedicine,* 2020, 196
- Insights into oscillometry: An Experimental Study for Improvement of Cuff-Based Blood Pressure Measurement Technology. **BOGATU, L. ; MUEHLSTEFF, J. ; BRESCH, E. ; SMINK, J. ; WOERLEE, P. H.** 2019 41st Annual International Conference of the IEEE Engineering in Medicine and Biology Society. EMBC, 2019, 7068-7071
- **MAAS JJ et al.** Estimation of mean systemic filling pressure in postoperative cardiac surgery patients with three methods. *Intensive Care Med.,* 2012, vol. 38, 1452-1460
- **AYA HD et al.** Transient stop-flow arm arterial-venous equilibrium pressure measurement: determination of precision of the technique. *J Clin Monit Comput.,* 2016, vol. 30 (1), 55-61
- **SEAGAR, A.D. et al.** Interpretation of venous occlusion plethysmographic measurements using simple model. *Med. Biol. Eng. Comput.,* 1984, vol. 22, 12
- **G. DRZEWIECKI.** *Theory of the Oscillometric Maximum and the Systolic and Diastolic Detection Ratios*